# EUROPÄISCHE PATENTSCHRIFT

(11) **EP 3 481 330 B1**
(45) Veröffentlichungstag und Bekanntmachung des Hinweises auf die Patenterteilung: **08.01.2020**
(21) Anmeldenummer: 18782332.3
(22) Anmeldetag: 20.09.2018
(51) Int. Cl.: A61C 19/04, A61C 7/36, A61C 7/12, A61C 7/08, G07C 3/02, A61C 7/30

(54) **SENSOR UND SYSTEM ZUR ÜBERWACHUNG DER TRAGEDAUER VON KIEFERORTHOPÄDISCHEN GUMMIZÜGEN**
SENSOR AND SYSTEM FOR MONITORING THE WEARING PERIOD OF ORTHODONTIC ELASTIC TRACTION DEVICES
CAPTEUR ET SYSTÈME DE SURVEILLANCE DE LA DURÉE DU PORT DES DISPOSITIFS ÉLASTIQUES ORTHODONTIQUES

(30) Priorität: 28.09.2017 EP 17193609
(43) Veröffentlichungstag der Anmeldung: 15.05.2019
(73) Patentinhaber: Otmar Kronenberg AG, 6003 Luzern (CH)
(72) Erfinder: KRONENBERG, Otmar, 6003 Luzern (CH)
(74) Vertreter: Koelliker, Robert
(86) Internationale Anmeldenummer: PCT/EP2018/075445
(87) Internationale Veröffentlichungsnummer: WO 2019/063405

(56) Entgegenhaltungen:
- WO-A1-2011/147985
- KR-A- 20170 004 401
- US-A1- 2006 166 157
- US-B1- 9 180 034

## Beschreibung

Die vorliegende Erfindung betrifft einen Sensor und ein System zur Überwachung der Tragedauer von kieferorthopädischen Gummizügen.

Zahnfehlstellungen werden in der Regel mittels sogenannten Zahnspangen korrigiert. Einerseits gibt es abnehmbare Zahnspangen, d.h. solche Spangen, die der Patient auf einfache Art und Weise selber entfernen und wieder einsetzen kann, beispielsweise Aligner. Andererseits gibt es sogenannt festsitzende Zahnspangen, welche nur durch den Behandler an den Zähnen angebracht und wieder entfernt werden können, sogenannte Multibracket-Apparaturen.

Zur Justierung der Position des Oberkiefers zum Unterkiefer kommen während einer Spangenkorrektur oft Gummizüge zum Einsatz, welche an festsitzenden Zahnspangen oder an Alignern befestigt werden. Dabei wird typischerweise am Ober- und am Unterkiefer je eine Multibracket-Apparatur und/oder ein Aligner angebracht. Die Multibracket-Apparaturen und Aligner weisen Vorrichtungen auf, an welchen der Gummizug auf einfache Art reversibel befestigt werden kann. Zur Justierung der Zahn- und Kieferstellung wird in der Regel der Gummizug an einem Bracket oder am Aligner im Oberkiefer und an einem Bracket oder am Aligner im Unterkiefer eingehängt. Durch den Gummizug entstehen intermaxilläre Kräfte, d.h. Kräfte zwischen dem Ober- und dem Unterkiefer, welche für die Justierung der Zahn- und Kieferstellung notwendig sind.

Solche Gummizüge können in aller Regel vom Patienten einfach entfernt und wieder angebracht werden. Um die gewünschte Justierung zu erzielen ist es zwingend, dass die Gummizüge gemäss den Vorgaben des Behandlers in der Regel über einen Zeitraum von täglich mehr als 6 Stunden getragen werden.

Um den Heilungserfolg zu überprüfen, muss der Patient regelmässig zur Kontrolle. Dabei kann es vorkommen, dass trotz Zahnspange mit Gummizug kein oder nur ein geringer Heilungsfortschritt beobachtet werden kann. In solchen Fällen weiss der Behandler nicht, ob dies medizinisch begründet ist, oder ob der Patient die geforderte Mindesttragedauer nicht eingehalten hat.

Die WO-A-03/096922 beschreibt ein kieferorthopädisches Bracket zur Verankerung einer festsitzenden kieferorthopädischen Apparatur an einem Zahn. Das Bracket umfasst eine zu verankernde Bracket-Basis und eine Bracket-Befestigung, wobei zwischen der Bracket-Basis und der Bracket- Befestigung eine Sensoreinrichtung angeordnet ist. Damit werden die auf die Zähne angewendeten Kräfte, Drücke und/oder Drehmomente objektiv erfasst und gemessen. Dadurch kann die kieferorthopädische Apparatur so eingestellt werden, dass optimale Kräfte und Drehmomente auf die Zähne des Patienten wirken um einen optimalen Heilungserfolg zu erzielen. Somit wird ein Bracket mit integriertem Sensor beschrieben. Nicht erwähnt ist jedoch ein von einem Bracket unabhängigen Sensor, der auf jede Multibracket-Apparatur befestigt werden kann. Zudem wird auch die Messung der intermaxillaren Kräfte mittels eines Gummizuges weder erwähnt noch vorgeschlagen.

Die WO-A-2011/147985 beschreibt ein System zur Überwachung der korrekten Verwendung eines intraoralen Geräts umfassend eine Stromquelle, einen Detektor zum Detektieren, wenn das intraorale Gerat im Mund zur Verwendung positioniert ist, einen Schreiber zum Aufzeichnen von Messdaten und ein Transponder zum Obermitteln der Messdaten. Das Überwachungssystem ist einstellbar auf eine bestimmte Eigenschaft eines Patienten oder einer Gruppe von Patienten, insbesondere bei behindernder Schlaf-Apnoe und Schnarchen. Das System eignet sich nicht zur Korrektur von Zahnfehlstellungen. Auch werden Multibracket-Apparaturen, Aligner sowie Gummizuge nicht erwähnt.

Die US-B-9,180,034 beschreibt eine Vorrichtung und ein Verfahren zur Unterstützung der Gewichtskontrolle. Dabei wird die Vorrichtung in den Mund eines Patienten zwischen gegenüberliegenden hinteren Backenzahnen eingeführt. Dadurch wird eine Erhöhung des Kauwiderstands erzielt, wodurch der Verzehr von Essen verlangsamt wird. Die Vorrichtung kann elastische Bänder, Magnete, Stoßdämpfer, Kombinationen davon und Sensoren zum Erfassen der Starke und Häufigkeit des Kauens umfassen. Das System eignet sich nicht zur Korrektur von Zahnfehlstellungen. Auch werden Multibracket-Apparaturen, Aligner und Timer nicht erwähnt und es wird keine Zeitspanne gemessen.

Die US-A-2006/0166157 offenbart ein orthodontischer Compliance-Monitor umfassend einen Sensor, welcher erfasst, wenn eine orthodontische Vorrichtung richtig positioniert ist, einen Prozessor, der eine Ausgabe des Sensors verarbeitet und Compliance-Daten erzeugt, sowie eine Speichervorrichtung zur Speicherung von Daten. Das System bezieht sich nur auf einen Kiefer und soll überprüfen, ob eine orthodontische Vorrichtung korrekt getragen wird. Daher eignet es sich nicht zum Justieren der Position des Oberkiefers zum Unterkiefer oder zur Kontrolle, ob ein Gummizug getragen wird oder nicht. Auch werden Multibracket-Apparaturen und Aligner für Ober- und Unterkiefer wie auch kieferorthopädische Gummizuge nicht erwähnt und es werden keine intermaxillaren Kräfte gemessen.

Daher ist es die Aufgabe der vorliegenden Erfindung, die Tragedauer von Gummizügen, welche bei kieferorthopädischen Behandlungen an festsitzenden Zahnspangen oder Alignern befestigt und zur Justierung der Position des Oberkiefers zum Unterkiefer während einer Spangenkorrektur eingesetzt werden, zu messen und zu dokumentieren, um objektive, und somit Patienten-unabhängige Angaben über die Tragedauer der Gummizüge zu erhalten.

Diese Aufgabe konnte überraschenderweise gelöst werden mit einem Sensor (1) zur Befestigung an einer Multibracket-Apparatur für den Oberkiefer (2), an einer Multibracket-Apparatur für den Unterkiefer (3), an einem Aligner für den Oberkiefer (2') und/oder an einem Aligner für den Unterkiefer (3'), um die Zeitspanne zu messen, während welcher eine Kraft auf einen Druck-, Zug- und/oder Scherkraftsensor (11) des Sensors (1) ausgeübt wird, wodurch die Tragedauer von kieferorthopädischen Gummizügen (4) für Multibracket-Apparaturen (2, 3) und/oder Aligner (2', 3') überwacht werden kann, wobei die Multibracket-Apparaturen eine Vielzahl von Brackets (23) aufweisen, umfassend den Druck-, Zug- und/oder Scherkraftsensor (11), einen Mikrokontroller mit Timer und Datenspeicher (12), wobei der Sensor (1)
- ein von der Multibracket-Apparatur (2, 3) und den Brackets (23) unabhängiger Sensor ist, sowie
- eine Befestigungsvorrichtung (15) zur Befestigung des Sensors (1) an einer Multibracket-Apparatur (2, 3) und/oder an einem Aligner (2', 3') aufweist, wobei der Sensor (1) anstelle der Befestigungsvorrichtung (15) auch an einem Aligner (2', 3') befestigt sein kann, und
- eine Gummizug-Halterung (16) und/oder Gummizug-Führung (17) umfasst,
wobei der Gummizug (4) an der Gummizug-Halterung (16) des Sensors (1) befestigbar und/oder der Gummizug (4) in der Gummizug-Führung (17) des Sensors (1) führbar ist, wodurch durch den Gummizug (4) eine Kraft auf den Druck-, Zug- und/oder Scherkraftsensor (11) des Sensors (1) wirkt und der Timer die Zeitspanne misst, während welcher die Kraft auf den Sensor (1) wirkt.

Beansprucht wird auch ein System (5) zur Überwachung der Tragedauer von kieferorthopädischen Gummizügen für Multibracket-Apparaturen (2, 3) und/oder Aligner (2', 3'), umfassend einen erfindungsgemässen Sensor (1), eine Multibracket-Apparatur oder einen Aligner für den Oberkiefer (2, 2') und eine Multibracket-Apparatur oder einen Aligner für den Unterkiefer (3, 3'), sowie einen Gummizug (4) und ein externes Lesegerät, wobei die Multibracket-Apparaturen (2, 3) eine Vielzahl von Brackets (23) und je einen Bogendraht (24) umfassen.

Der erfindungsgemässe Sensor (1) und das erfindungsgemässe System (5) weisen überraschenderweise eine Vielzahl von Vorteilen auf. So kann die Zeit, während welcher der Patient die Gummizüge trägt, d.h. die Tragedauer der Gummizüge durch den Patienten, auf einfache Art und Weise gemessen werden, und somit auch die Zeit, während welcher eine korrigierende Kraft auf die Zähne und den Ober- und den Unterkiefer ausgeübt wird. Somit muss sich der Behandler nicht mehr auf die Aussage des Patienten abstützen, sondern er hat mit der vorliegenden Erfindung ein neutrales Beurteilungsmittel zur Überprüfung der Tragedauer der kieferorthopädischen Gummizüge (4) mittels Messung der Zeitspanne, während welcher eine Kraft auf den Sensor (11) ausgeübt wird.

Ist der Sensor (1) an einer Multibracket-Apparatur (2, 3) und/oder an einem Aligner (2', 3') befestigt, kann der Gummizug (4) - wenn er getragen wird - vom Oberkiefer zum Unterkiefer, und somit von einer zur anderen Multibracket-Apparatur (2, 3), gespannt werden. Gleiches gilt bei Alignern (2', 3'). Dabei kann der Gummizug (4) an der Gummizug-Halterung (16) des Sensors (1) befestigt und/oder in der Gummizug-Führung (17) des Sensors (1) geführt werden. Dadurch übt der Gummizug (4) eine Kraft auf den Druck-, Zug- und/oder Scherkraftsensor (11) des Sensors (1) aus. Sobald und solange nun eine Kraft, insbesondere durch den Gummizug (4), auf den Druck-, Zug- und/oder Scherkraftsensor (11) wirkt, wird dies durch den Timer gemessen und die Zeitspanne kann festgestellt werden, während welcher eine Kraft auf den Druck-, Zug- und/oder Scherkraftsensor (11) des Sensors (1) wirkt. Mit anderen Worten: Sobald der Gummizug (4) eine Kraft auf den Druck-, Zug- und/oder Scherkraftsensor (11) ausübt, wird - einem ON/OFF-Schalter gleich - mittels Timer die Zeitspanne gemessen, bis der Gummizug (4) entfernt wird und keine Kraft mehr auf den Sensor (11) ausgeübt wird. Dann wird auch der Timer gestoppt und die gemessene Zeitspanne beendet. Werden mehrere Zeitspannen hintereinander gemessen, können diese auch addiert werden.

Da der erfindungsgemässe Sensor (1) nicht in einem Bracket (23) integriert ist - und somit Bracket-unabhängig ist - kann der Sensor (1) im Wesentlichen an jeder Multibracket-Apparatur (2, 3) und an jedem Aligner (2', 3') befestigt werden. Mit anderen Worten: Der erfindungsgemässe Sensor (1) kann auf einfache Art und Weise an jeder geeigneten Stelle jeder Multibracket-Apparatur (2, 3) oder an jedem Aligner (2', 3') aller Hersteller befestigt werden. Somit ist das erfindungsgemässe System (5) vorteilhafterweise nicht auf Multibracket-Apparaturen (2, 3) eines oder einiger wenigen Hersteller limitiert, sondern kann jegliche Multibracket-Apparatur (2, 3) umfassen.

Zudem kann der Sensor (1) auch grössere Dimensionen als ein einzelnes Bracket (23) aufweisen, wodurch ein Sensor (1) hergestellt werden kann, welcher robust ist und den geforderten Anforderungen, auch beispielsweise in Bezug auf die Grösse der Batterie und des Funkmoduls, in allen Belangen genügen kann. So ist beispielsweise die Grösse des Sensors (1) nicht auf eine Zahnoberfläche limitiert, wie dies bei Brackets (23) der Fall ist, sondern der Sensor (1) kann sich problemlos auch über zwei oder mehr Zahnoberflächen des gleichen Kiefers erstrecken.

Vor dem Essen und der Zahnreinigung wird vorteilhafterweise - und in aller Regel - der Gummizug (4) entfernt. Dabei ist es wichtig, dass nach dem Essen und nach der Zahnreinigung der Gummizug (4) wieder an den Häkchen der Brackets (23) befestigt wird, um die zur erfolgreichen Therapie notwendigen Kräfte zu erzeugen.

Erfindungsgemäss wird unter dem Begriff Multibracket-Apparaturen (2, 3) - auch festsitzende Zahnspange genannt - eine festsitzende kieferorthopädische Apparatur verstanden, welche im Wesentlichen aus einer Vielzahl von - in der Regel unterschiedlichen - Brackets (23) und einem Bogendraht (24) besteht. Die Brackets (23) werden durch den Behandler an der Aussenseite einer Vielzahl von Zähnen mindestens eines Kiefers geklebt. Anschliessend wird ein Bogendraht (24), auch Behandlungsbogen genannt, entlang den Brackets (23) geführt. Im Laufe der Behandlung wird der Bogendraht (24) mehrmals geändert, wobei der jeweils neu eingesetzte Bogendraht (24) eine zunehmende Dimension und Rigidität aufweist, wodurch die Zähne bewegt werden. Solche Multibracket-Apparaturen mit Brackets (23) und Bogendraht (24) sind dem Fachmann bekannt und im Handel erhältlich.

Unter Brackets (23) werden erfindungsgemäss alle Arten von Brackets verstanden, welche bei der Herstellung von Multibracket-Apparaturen Anwendung finden können. Die jeweilige Art und Form der Brackets (23) wird je nach Zahn und dessen zu korrigierender Stellung individuell ausgewählt. So können Brackets (23) auch Häkchen zum Befestigen von Gummizügen aufweisen. Erfindungsgemäss umfasst der Begriff Bracket (23) auch Tubes oder Bänder. Die Brackets können aus Stahl, insbesondere Edelstahl, Keramik oder Kunststoff gefertigt sein. Geeignete Brackets sind dem Fachmann bekannt und im Handel erhältlich.

Als Aligner (2', 3') werden erfindungsgemäss durchsichtige Kunststoffschienen verstanden, welche für die jeweilige Zahnstellung individuell gefertigt werden. Die Aligner sind in der Regel durchsichtig und können von Aussenstehenden kaum wahrgenommen werden. Bei Bedarf ist es in aller Regel möglich, am Aligner eine Gummizug-Halterung zu befestigen, oder bei der Herstellung des Aligners eine solche im Aligner zu integrieren. Hierzu kann beispielsweise eine Kerbe im Aligner (2', 3') angebracht werden. Alternativ kann auch eine Befestigungsmöglichkeit, beispielsweise ein Knöpfchen oder Button, direkt auf den Zahn geklebt werden, wobei der Aligner (3') an dieser Stelle eine Aussparung aufweist. Geeignete Befestigungsmöglichkeiten wie Knöpfchen oder Buttons sind dem Fachmann bekannt und im Handel erhältlich. Im Gegensatz zur festsitzenden Multibracket-Apparatur können Aligner vom Patienten selber herausgenommen werden, d.h. sie sind abnehmbar.

Unter dem Begriff Gummizug (4) wird erfindungsgemäss jedes geeignete Mittel verstanden, welches Zahnärzte bei Multibracket-Apparaturen (2, 3) resp. Alignern (2', 3') verwenden, um eine Kraft auf den Oberkiefer und/oder Unterkiefer auszuüben. Somit kann der Gummizug (4) auch aus einem anderen Material gefertigt sein als Gummi. Der Gummizug (4) ist typischerweise ein Gummiband, welches sich unter Kraftaufwand reversibel dehnen lässt. Besonders bevorzugt liegt der Gummizug (4) in Form von Gummiringen vor, d.h. in Form eines kurzen Stücks Gummi in Ringform. Mit dem Gummizug (4) wird die Multibracket-Apparatur (2) resp. der Aligner (2') des Oberkiefers mit der Multibracket-Apparatur (3) resp. der Aligner (3') des Unterkiefers verbunden. Dabei wird der Gummizug (4) bevorzugt an einem Bracket (23), am Bogendraht (24), an einem Zahn, an einem im Aligner (2', 3') integrierten Häkchen und/oder am Sensor (1), insbesondere an der Gummizug-Halterung (16) des Sensors (1), befestigt.

### Der Sensor (1)

Der erfindungsgemässe Sensor (1) eignet sich insbesondere zur Befestigung an einer Multibracket-Apparatur für den Oberkiefer (2), an einer Multibracket-Apparatur für den Unterkiefer (3), an einem Aligner für den Oberkiefer (2') und/oder an einem Aligner für den Unterkiefer (3'), um die Zeitspanne zu messen, während welcher eine Kraft auf einen Druck-, Zug- und/oder Scherkraftsensor (11) des Sensors (1) ausgeübt wird, wodurch die Tragedauer von kieferorthopädischen Gummizügen (4) für festsitzende Multibracket-Apparaturen (2, 3) und/oder abnehmbare Aligner (2', 3') überwacht, d.h. gemessen und gespeichert, werden kann. Zusätzlich kann der Sensor (1) beispielsweise auch zur Messung der durch den Gummizug (4) ausgeübten Kraft, d.h. Druck-, Zug- und/oder Scherkraft, - in Funktion der Tragedauer - eingesetzt werden. Die Zeitspanne wird mit einem im Sensor integrierten Timer gemessen. Pro Patient kann ein oder mehr als ein Sensor (1) gleichzeitig verwendet werden.

Der erfindungsgemässe Sensor (1) ist ein von der Multibracket-Apparatur (2, 3) und den Brackets (23) unabhängiger Sensor, d.h. der Sensor (1) interagiert weder mit der Multibracket-Apparatur (2, 3), den Brackets (23) noch mit dem Aligner (2', 3'). Der Sensor (1) umfasst einen Druck-, Zug- und/oder Scherkraftsensor (11), einen Mikrokontroller mit Timer und Datenspeicher (12), eine Befestigungsvorrichtung (15) zur Befestigung des Sensors (1) an einer Multibracket-Apparatur (2, 3) und/oder an einem Aligner (2', 3'), sowie eine Gummizug-Halterung (16) und/oder Gummizug-Führung (17). Dabei ist der Gummizug (4) an der Gummizug-Halterung (16) des Sensors (1) befestigbar und/oder der Gummizug (4) ist in der Gummizug-Führung (17) des Sensors (1) führbar, wodurch durch den Gummizug (4) eine Kraft auf den Druck-, Zug- und/oder Scherkraftsensor (11) des Sensors (1) ausübbar ist. Somit sind der Druck-, Zug- und/oder Scherkraftsensor (11) sowie die Gummizug-Halterung (16) und/oder die Gummizug-Führung (17) des Sensors (1) so angeordnet, dass beim Tragen des Gummizugs (4) die vom Gummizug (4) ausgeübte Kraft auf den Druck-, Zug- und/oder Scherkraftsensor (11) übertragen wird. Die Gummizug-Halterung (16) umfasst zudem typischerweise mindestens eine Vorrichtung, beispielsweise ein Häkchen, zum Befestigen von Gummizügen.

Der Timer misst die Zeitspanne, während welcher Kraft auf den Sensor (1) ausgeübt wird. Dabei kann der Timer als Software oder als Hardware realisiert sein. Geeignete Timer sind dem Fachmann bekannt und er kann die geeignete Auswahl treffen.

Der Sensor (1) kann anstelle der Befestigungsvorrichtung (15) auch an einem Aligner (2', 3') befestigt, insbesondere geklebt und/oder innerhalb des Aligners angeordnet, d.h. platziert, sein. So kann der Sensor (1) beispielsweise mit dem Kunststoff des Aligners (2', 3') so umschlossen sein, dass der Sensor (1) am oder im Aligner (2', 3') fixiert ist. Diese Ausführungsform wird vorteilhafterweise während der Herstellung des Aligners (2', 3') erstellt. Alternativ kann der Behandler, beispielsweise der Zahnarzt, den Sensor (1) am oder im Aligner (2', 3') befestigen - beispielsweise mittels Kleben und/oder Stanzen. Somit ist der Sensor (1) unabhängig vom Aligner (2', 3') und der Sensor (1) interagiert nicht mit dem Aligner (2', 3').

Der Mikrokontroller mit Datenspeicher und Timer (12) des Sensors (1) ermöglicht und überwacht u.a. das Ein- und Auslesen der im Druck-, Zug- und/oder Scherkraftsensor (11) erfassten Daten in den und aus dem Datenspeicher des Mikrokontrollers (12). Mit Hilfe der Taktfrequenz des Mikrokontrollers kann typischerweise auch der Timer in Form einer Software oder Hardware gesteuert werden. Zudem können mit Hilfe des Mikrokontrollers Rechenoperationen mit den erfassten und/oder gespeicherten Daten durchgeführt werden. Dabei kann der Mikrokontroller Teil des Druck-, Zug- und/oder Scherkraftsensors (11) und/oder des Datenspeichers sein. Der Mikrokontroller (12) kann auch - alternativ oder zusätzlich - im Sensor (1) als separates, d.h. vom Datenspeicher unabhängiges, Bauteil vorliegen. Erfindungsgemäss werden sie jedoch zusammen als Mikrokontroller mit Timer und Datenspeicher (12), als Mikrokontroller mit Datenspeicher (12) oder auch nur Mikrokontroller (12) oder Datenspeicher (12) genannt, unabhängig, ob sie in Form von einem Bauteil oder als mehrere Bauteile vorliegen.

Der Sensor (1) umfasst zudem in einer bevorzugten Ausführungsform ein Gehäuse (13), eine Stromversorgung (14), insbesondere eine Batterie, und/oder eine Datenübertragungsvorrichtung (18), um die gemessenen und im Sensor (1) - insbesondere im Datenspeicher (12) - gespeicherten Daten auszulesen, d.h. zu einem anderen Medium, wie beispielsweise zu einem Computer, zu übertragen. Dabei umfasst die Datenübertragungsvorrichtung (18) bevorzugt einen Funksender und/oder eine Steckverbindung. Das Auslesen der Daten kann regelmässig - beispielsweise täglich - beispielsweise durch den Patienten und/oder automatisiert mittels Funkübermittlung, erfolgen. Oft ist es jedoch bevorzugt, dass das Auslesen bei der periodischen Kontrolle beim Behandler erfolgt. Das Auslesen kann beispielsweise mittels Funk, insbesondere mittels NFC, RFID und/oder Bluetooth, und/oder mittels Steckverbindung zu einem externen Lesegerät übertragen werden. Dazu kann der Sensor (1) an der Multibracket-Apparatur (2, 3) resp. am Aligner (2', 3') befestigt bleiben, oder er kann zum Auslesen entfernt werden und gegebenenfalls anschliessend wieder an der Multibracket-Apparatur (2, 3) resp. am Aligner (2', 3') fixiert werden.

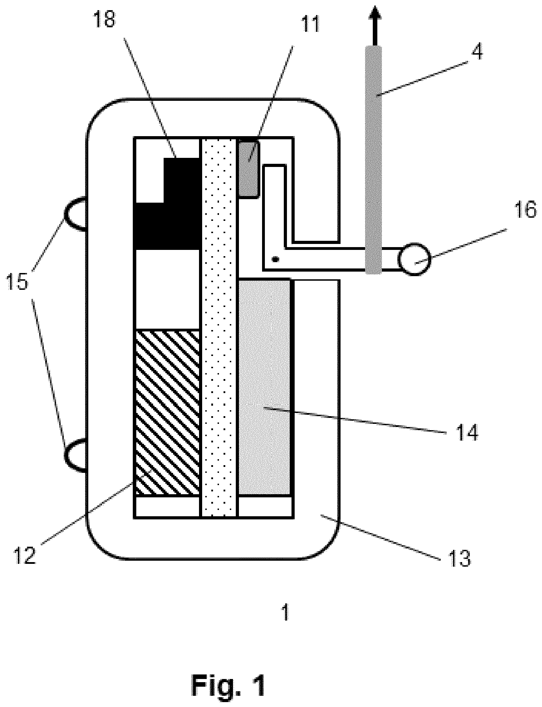

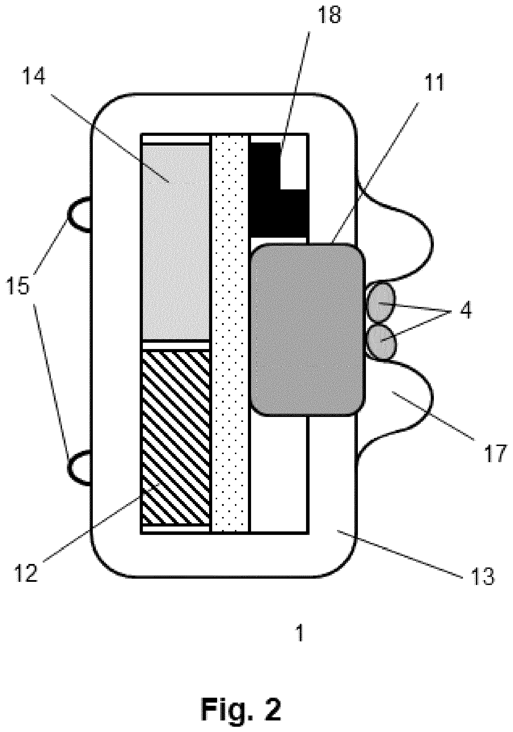

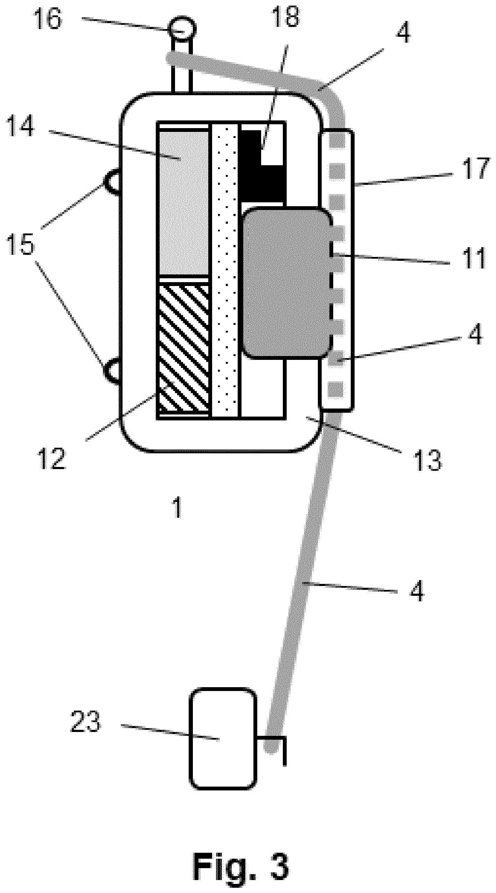

In einer anderen bevorzugten Ausführungsform des Sensors (1) kann ein Gummizug (4) an der Gummizug-Halterung (16) des Sensors (1) reversibel befestigt werden um die Gummizug-Halterung (16) mit dem Gummizug (4) mit einem Bracket (23), Bogendraht (24) oder Aligner (2', 3') zu verbinden. Dabei übt die Gummizug-Halterung (16) bei gespanntem Gummizug (4) gegebenenfalls - insbesondere bevorzugt - eine Kraft auf den Druck-, Zug- und/oder Scherkraftsensor (11) des Sensors (1) aus, wobei im Sensor (1) zumindest die Zeiten, während derer diese Kraft ausgeübt wurde, gemessen und gespeichert werden. Eine nicht-limitierende Ausführungsform ist in Fig. 1 dargestellt. Alternativ - oder in Kombination - kann ein Gummizug (4) in der Gummizug-Führung (17) des Sensors (1) geführt werden, wodurch eine Kraft auf den Druck-, Zug- und/oder Scherkraftsensor (11) ausgeübt wird, wenn der Gummizug (4) von einem Bracket (23) der Multibracket-Apparatur des Oberkiefers (2) oder vom Aligner des Oberkiefers (2') zu einem Bracket (23) der Multibracket-Apparatur des Unterkiefers (3) oder zum Aligner des Unterkiefers (3') gespannt ist. Eine solche beispielhafte Anordnung ist in Fig. 2 dargestellt. Dabei kann gegebenenfalls, insbesondere bevorzugt, der Gummizug (4) anstelle an einem Bracket (23) oder Aligner (2', 3') auch an der Gummizug-Halterung (16) des Sensors (1) befestigt sein, wie in Fig. 3 gezeigt.

In einer bevorzugten Ausführungsform umfasst die Befestigungsvorrichtung (15) des Sensors (1) eine Öse, Klemme, Schraube, ein Draht, Gummizug, Steckverbindung, Elastik und/oder Klebefläche, anhand welcher der Sensor (1) im Wesentlichen an jeder beliebigen Multibracket-Apparatur (2, 3) und an jeden beliebigen Aligner (2', 3') befestigt werden. Dem Fachmann sind solche Befestigungsmittel und Befestigungsarten bekannt und er kann damit den erfindungsgemässen Sensor (1) an der Multibracket-Apparatur (2, 3) und am Aligner (2', 3') korrekt und genügend befestigen.

### Das System (5)

Das erfindungsgemässe System (5) ist insbesondere zur Überwachung, d.h. zum Messen und Speichern, der Tragedauer von kieferorthopädischen Gummizügen für Multibracket-Apparaturen (2, 3) und/oder Aligner (2', 3') geeignet. Überraschenderweise können im erfindungsgemässen System Brackets, Multibracket-Apparaturen, Aligner und Gummizüge von beliebigen Herstellern eingesetzt werden, sofern sie sich für kieferorthopädische Anwendungen eignen. Das System (5) umfassend den Sensor (1) kann zudem auch zur Messung der durch den Gummizug (4) ausgeübten Kraft - in Funktion der Tragedauer - eingesetzt werden.

Das System (5) umfasst einen erfindungsgemässen Sensor (1), eine Multibracket-Apparatur oder einen Aligner für den Oberkiefer (2, 2') und eine Multibracket-Apparatur oder einen Aligner für den Unterkiefer (3, 3'), sowie einen Gummizug (4) und ein externes Lesegerät, wobei die Multibracket-Apparaturen (2, 3) eine Vielzahl von Brackets (23) und je einen Bogendraht (24) umfassen. Dabei kann pro Patient ein Sensor (1), welcher am Ober- oder Unterkiefer des Patienten befestigt wird, genügen. Es ist aber auch möglich, dass beim Patienten zwei oder mehr Sensoren (1) eingesetzt werden, typischerweise pro eingesetzten Gummizug (4) ein Sensor (1). Bei Bedarf ist es auch möglich, sowohl am Oberkiefer wie auch am Unterkiefer einen Sensor (1) zu befestigen. Dabei umfasst vorteilhafterweise mindestens ein Bracket (23) der Multibracket-Apparatur oder des Aligners für den Oberkiefer (2, 2') oder der Multibracket-Apparatur oder des Aligners für den Unterkiefer (3, 3') eine Anordnung - insbesondere ein Häkchen, zum Befestigen von Gummizügen.

Im System (5) kann der Sensor (1) mittels der Befestigungsvorrichtung (15), welche mit dem Sensor (1) typischerweise starr verbunden ist, an einem oder mehreren Brackets (23) jeder beliebigen Multibracket-Apparatur (2, 3) sowie an jeden beliebigen Aligner (2', 3') befestigt werden. Es ist auch möglich, dass der Sensor (1) zusätzlich - oder nur - am Bogendraht (24) befestigt wird. Alternativ - oder in Kombination - kann er auch an einem oder mehreren Zähnen befestigt werden.

In einer bevorzugten Ausführungsform des Systems (5) kann die Befestigungsvorrichtung (15) des Sensors (1) mit mindestens einem Bracket (23) und/oder dem Bogendraht (24) einer Multibracket-Apparatur (2, 3), und/oder einem Aligner (2', 3'), mit einem Befestigungsmittel verbunden werden.

Geeignete - und oft auch bevorzugte - Befestigungsmittel zur Befestigung der Befestigungsvorrichtung (15) des Sensors (1) an einer Multibracket-Apparatur (2, 3) und/oder an einem Aligner (2', 3') umfassen einen Draht, eine Schraube, eine Klemme, einen Gummizug, einen Elastik, einen Kunststoff und/oder einen Klebstoff oder Klebstoffkombination. Dabei werden typischerweise die Befestigungsmittel mit der Befestigungsvorrichtung (15) des Sensors (1) abgestimmt, um eine geeignete Befestigung zu erhalten. Der Fachmann weiss, welche Befestigungsmittel für die spezifische Anwendung geeignet sind und welche Befestigungsmittel er mit welcher Befestigungsvorrichtung (15) optimal kombinieren kann. Wird zudem der Sensor (1) an einen Aligner befestigt, ist es auch möglich, den Sensor (1) direkt während der Herstellung des Aligners (2', 3') mit dem Sensor zu verbinden.

In einer bevorzugten Ausführungsform des erfindungsgemässen Systems (5) ist der erfindungsgemässe Sensor (1), wenn er an einer Multibracket-Apparatur (2, 3) befestigt ist, i) zwischen zwei Brackets (23) der gleichen Multibracket-Apparatur (2, 3) angeordnet, und/oder ii) erstreckt er sich über mindestens ein, bevorzugt über mindestens zwei, Brackets (23) der gleichen Multibracket-Apparatur (2, 3).

In einer anderen bevorzugten Ausführungsform des erfindungsgemässen Systems (5) ist der erfindungsgemässe Sensor (1), wenn er an einem Aligner (2', 3') befestigt ist, i) im seitlichen Aussenbereich des Aligners (2', 3') angeordnet, und/oder ii) erstreckt er sich über mindestens einen, bevorzugt über mindestens zwei, Zahnkronen des gleichen Aligners (2', 3').

### Das Verfahren

Das nicht erfindungsgemässe Verfahren eignet sich besonders zum Überwachen, d.h. zum Messen und Speichern, der Tragedauer von kieferorthopädischen Gummizügen an Multibracket-Apparaturen (2, 3) und/oder Aligner (2', 3') mit dem erfindungsgemässen System (5). Zusätzlich kann das Verfahren beispielsweise auch zur Messung der durch den Gummizug (4) ausgeübten Kraft - in Funktion der Tragedauer - eingesetzt werden.

In einem ersten Schritt i) des nicht erfindungsgemässen Verfahrens wird der erfindungsgemässe Sensor (1) - wie vorgängig beschrieben - an einer Multibracket-Apparatur (2, 3) und/oder an einem Aligner (2', 3'), typischerweise mit einem Befestigungsmittel mittels der Befestigungsvorrichtung (15), befestigt. Dabei erfolgt die Befestigung des Sensors (1) an der Multibracket-Apparatur (2, 3) in der Regel nachdem die Multibracket-Apparatur an den Zähnen befestigt wurde. Die Befestigung des Sensors (1) an den Aligner (2', 3') kann während oder nach der Herstellung des Aligners (2', 3') erfolgen. Wird der Sensor (1) nach der Herstellung des Aligners (2', 3') am Aligner (2', 3') befestigt, kann die Befestigung des Sensors (1) an den Aligner (2', 3') ausserhalb des Mundes oder direkt im Mund erfolgen. Dieser Schritt erfolgt in aller Regel beim Behandler.

In einem zweiten Schritt ii) des nicht erfindungsgemässen Verfahrens wird der Gummizug (4) von einer Multibracket-Apparatur (2, 3) und/oder von einem Aligner (2', 3') zur anderen Multibracket-Apparatur (2, 3) und/oder Aligner (2', 3') gespannt, wobei der Gummizug (4) an der Gummizug-Halterung (16) des Sensors (1) befestigt und/oder der Gummizug (4) in der Gummizug-Führung (17) des Sensors (1) geführt wird, wodurch durch den Gummizug (4) eine Kraft auf den Druck-, Zug- und/oder Scherkraftsensor (11) des Sensors (1) ausgeübt wird. Dieser Schritt erfolgt zunächst direkt im Anschluss an Schritt i) beim Behandler und dann regelmässig, d.h. typischerweise mehrmals täglich, durch den Patienten selber.

In einem weiteren Schritt iii) des nicht erfindungsgemässen Verfahrens wird mit dem Timer mindestens die Zeitspanne der auf den Druck-, Zug- und/oder Scherkraftsensor (11) ausgeübten Kraft gemessen, d.h. die Zeitspanne, während welcher die durch den Gummizug (4) erzeugte Kraft auf den Druck-, Zug- und/oder Scherkraftsensor (11) ausgeübt wird. Dabei wird die mit dem Timer gemessene Zeitspanne auf dem Datenspeicher des Mikrokontrollers (12) vom Sensor (1) gespeichert. Dieser Schritt erfolgt immer dann, wenn der Gummizug (4) an der Multibracket-Apparatur (2, 3) und/oder am Aligner (2', 3') befestigt ist.

In einem weiteren Schritt vi) des nicht erfindungsgemässen Verfahrens werden die im Datenspeicher des Mikrokontrollers (12) des Sensors (1) gespeicherten Daten unter Verwendung des Lesegeräts ausgelesen. Dabei kann als Lesegerät ein spezifisches, beispielsweise für diesen Verfahrensschritt optimiertes Lesegerät, zur Anwendung kommen. Alternativ kann als Lesegerät auch ein Computer und/oder Mobiltelefon wie Smartphone dienen, welcher die Daten vom Datenspeicher (12) des Sensors (1) auslesen kann. Dabei erfolgt das Auslesen der Daten typischerweise mit üblichen Methoden, die dem Fachmann bekannt sind. Dieser Schritt erfolgt in aller Regel bei der Kontrolle und beim Nachjustieren beim Behandler. Alternativ - oder zusätzlich - kann das Auslesen der Daten auch in kürzeren Zeitabständen, beispielsweise täglich, erfolgen. Dabei werden die Daten bevorzugt per Funk, und gegebenenfalls mittels Internet, zum Lesegerät gesendet. Das Lesegerät kann sich beispielsweise in der Behandler-Praxis befinden und/oder an einem anderen Ort, beispielsweise an einem zentralen Ort, von welchem aus die gesammelten Daten zu den einzelnen Behandler-Praxen weitergeleitet werden.

In einer bevorzugten Ausführungsform des nicht erfindungsgemässen Verfahren werden die im Datenspeicher (12) des Sensors (1) gespeicherten Daten mittels Funk, insbesondere mittels NFC, RFID und/oder Bluetooth, und/oder mittels Steckverbindung, d.h. mittels Kabelverbindung, zum externen Lesegerät übertragen und gegebenenfalls gespeichert, weiterverarbeitet und/oder ausgedruckt.

### Die Verwendung

Der erfindungsgemässe Sensor (1), das erfindungsgemässe System (5) und das nicht erfindungsgemässen Verfahren eignen sich zu nicht erfindungsgemässen Verwendung mit kieferorthopädischen Apparaturen, insbesondere Multibracket-Apparaturen (2, 3) und Alignern (2', 3') für Oberkiefer und Unterkiefer.

Eine besonders bevorzugte nicht erfindungsgemässe Verwendung umfasst das Messen der Zeitspanne, und somit der Tragedauer, während welcher eine Kraft auf einen Druck-, Zug- und/oder Scherkraftsensor (11) des Sensors (1) ausgeübt wird, zum Überwachen - und somit das Messen und Speichern - der Tragedauer von kieferorthopädischen Gummizügen an Multibracket-Apparaturen und Alignern.

Der Sensor (1), das Systems (5) und das Verfahrens können aber auch - insbesondere zusätzlich zum Überwachen der Tragedauer - nicht erfindungsgemäss verwendet werden zur Messung der durch den Gummizug (4) ausgeübten Kraft, insbesondere der ausgeübten Kraft in Funktion der Tragedauer.

Es werden folgende Bezugszeichen verwendet:
- 1: Sensor
- 2: Multibracket-Apparatur für den Oberkiefer
- 3: Multibracket-Apparatur für den Unterkiefer
- 2': Aligner für den Oberkiefer
- 3': Aligner für den Unterkiefer
- 4: Gummizug
- 5: System
- 11: Druck-, Zug- und/oder Scherkraftsensor
- 12: Mikrokontroller mit Timer und Datenspeicher
- 13: Gehäuse
- 14: Stromversorgung
- 15: Befestigungsvorrichtung
- 16: Gummizug-Halterung
- 17: Gummizug-Führung
- 18: Datenübertragungsvorrichtung
- 23: Vielzahl von Brackets
- 24: Bogendraht

Im Folgenden werden nicht-limitierende, bevorzugte Ausführungsformen des erfindungsgemässen Sensors (1) und des erfindungsgemässen Systems (5) anhand der nachfolgenden Zeichnungen beschrieben. Diese sind nicht einschränkend auszulegen und werden als Bestandteil der Beschreibung verstanden:
- Fig. 1: zeigt beispielhaft eine nicht-limitierende Ausführungsform des erfindungsgemässen Sensors (1). In der Mitte des Gehäuses (13) ist eine Leiterplatte (schwach gemustert dargestellt) angeordnet, an welcher beispielhaft der Mikrokontroller mit Timer und Datenspeicher (12), als Stromversorgung (14) eine Batterie, als Datenübertragungsvorrichtung (18) ein Transmitter sowie einen Druck-, Zug- und/oder Scherkraftsensor (11). Der Mikrokontroller mit Timer und Datenspeicher (12), die Stromversorgung (14), Datenübertragungsvorrichtung (18) und der Druck-, Zug- und/oder Scherkraftsensor (11) sind über die Leiterplatte miteinander so verbunden, dass die gewünschten Informationen ausgetauscht werden können und dass die Stromversorgung der einzelnen Komponenten gewährleistet ist. An der Gummizug-Halterung (16) des Sensors (1) ist ein Gummizug (4) angebracht. Dieser erstreckt sich nun von der einen MultibracketApparatur (2, 3), an welcher der Sensor (1) befestigt ist, in Richtung - mit dem Pfeil angedeutet - eines Häkchens eines Brackets (23) der anderen Multibracket-Apparatur (2, 3). Wird nun der Gummizug korrekterweise zwischen den beiden Multibracket-Apparaturen (2, 3) gespannt, wird auf die Gummizug-Halterung (16) des Sensors (1) ein Zug ausgeübt. Durch die L-förmige Anordnung der GummizugHalterung (16) mit Drehpunkt beim Ecken der Gummizug-Halterung (16) entsteht nun ein Druck auf den Sensor (11). Dieser Druck triggert - einem ON/OFF-Schalter gleich - den Timer, wodurch die Zeitdauer gemessen wird, während welcher der Gummizug den Druck ausübt. Die gemessenen Informationen werden auf dem Datenspeicher (12) gespeichert und gegebenenfalls weiterverarbeitet.
An der Aussenseite des Gehäuses (13), gegenüber der Gummizug-Halterung (16) sind Ösen als Befestigungsvorrichtung (15) des Sensors (1) angeordnet, mit welcher der Sensor (1) an der Multibracket-Apparatur (2, 3) fixiert werden kann.
- Fig. 2: zeigt beispielhaft eine weitere, nicht-limitierende Ausführungsform des erfindungsgemässen Sensors (1). Innerhalb des Gehäuses (13) ist der Mikrokontroller mit Timer und Datenspeicher (12), die Stromversorgung (14), die Datenübertragungsvorrichtung (18) sowie der Druck-, Zug- und/oder Scherkraftsensor (11) angeordnet. Diese sind über die Leiterplatte (schwach gemustert) so miteinander verbunden, dass die gewünschten Informationen ausgetauscht werden können und dass die Stromversorgung der einzelnen Komponenten gewährleistet ist.
An einer Aussenseite des Gehäuses (13) sind Ösen als Befestigungsvorrichtung (15) des Sensors (1) angeordnet, mit welcher der Sensor (1) an der Multibracket-Apparatur (2, 3) fixiert werden kann.
Auf der Seite des Gehäuses (13), die der Befestigungsvorrichtung (15) gegenüber liegt, ist - beispielhaft und im Querschnitt gezeigt - eine Gummizug-Führung (17) des Sensors (1) angeordnet, in welcher der Gummizug (4) liegt und Druck auf den Druck-, Zug- und/oder Scherkraftsensor (11) ausübt. Um die Empfindlichkeit des Sensors (11) zu erhöhen, reicht dieser beispielhaft etwas über die Abmessungen des Gehäuses (13) hinaus, sodass der Gummizug (4) einen etwas erhöhten Druck auf den Sensor (11) ausüben kann. Dazu ist der Gummizug (4) typischerweise an je einem Bracket (23), insbesondere an einem Häkchen eines Brackets (23), der Multibracket-Apparatur für den Oberkiefer (2) und der Multibracket-Apparatur für Unterkiefer (3) befestigt (nicht dargestellt).
- Fig. 3: zeigt beispielhaft eine andere, nicht-limitierende Ausführungsform des erfindungsgemässen Sensors (1). Analog der in Fig. 2 gezeigten Darstellung wird der Gummizug (4) durch die Gummizug-Führung (17) geführt, wobei jedoch der Gummizug (4) an der Gummizug-Halterung (16) des Sensors (1) befestigt ist und innerhalb der Gummizug-Führung (17) zu einem Bracket (23) der anderen Multibracket-Apparatur geführt wird, wo der Gummizug (4) an einem Häkchen befestigt ist.
Der Druck-, Zug- und/oder Scherkraftsensor (11) reicht wie in Fig. 2 beispielhaft etwas über die Abmessungen des Gehäuses (13) hinaus, sodass der Gummizug (4) einen etwas erhöhten Druck auf den Sensor (11) ausüben kann. Sobald ein erhöhter Druck gemessen wird, triggert dies den Timer, bis der Druck entfernt wird, d.h. bis der Gummizug (4) entfernt wird. Dadurch wird die Zeitspanne gemessen, während welcher eine Kraft auf den Druck-, Zug- und/oder Scherkraftsensor (11) des Sensors (1) ausgeübt wird, wodurch die Tragedauer von kieferorthopädischen Gummizügen (4) für Multibracket-Apparaturen (2, 3) und/oder Aligner (2', 3') überwacht werden kann.

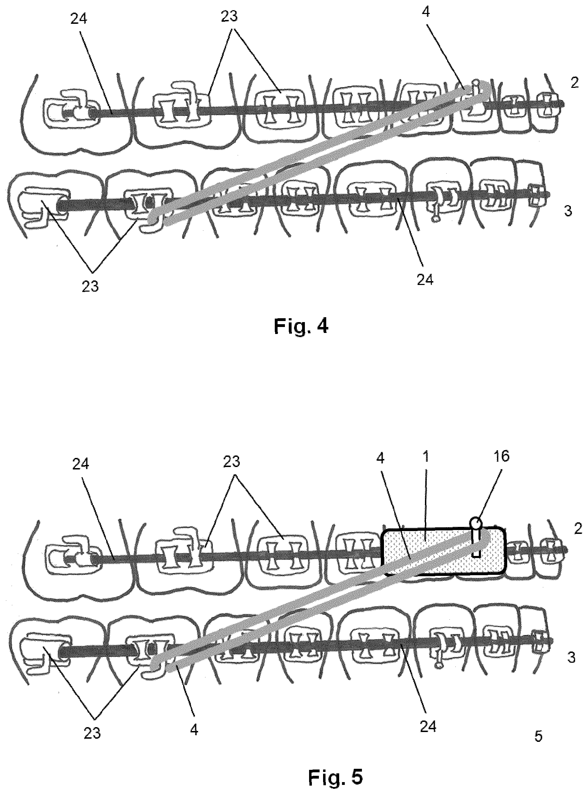

- Fig. 4: zeigt eine heute üblicherweise verwendete Multibracket-Apparatur für den Oberkiefer (2) und eine Multibracket-Apparatur für den Unterkiefer (3) mit jeweils einer Vielzahl von unterschiedlichen Brackets (23) ohne den erfindungsgemässen Sensor (1) und stellt somit den Stand der Technik dar. Einige der Brackets (23), die mit dem Bogendraht (24) miteinander verbunden sind, weisen ein Häkchen auf, an welchen beispielsweise ein Gummizug (4) befestigt werden kann. Vor dem Essen und der Zahnreinigung wird vorteilhafterweise der Gummizug (4) entfernt. Dabei ist es wichtig, dass nach dem Essen und nach der Zahnreinigung der Gummizug (4) wieder an den Häkchen der Brackets (23) befestigt wird, um die zur erfolgreichen Therapie notwendigen Kräfte zu erzeugen. Mit diesem heutzutage vielfach eingesetzten System kann jedoch nicht überprüft werden, ob die Gummizüge auch tatsächlich getragen werden.
- Fig. 5: zeigt das erfindungsgemässe System (5) umfassend den erfindungsgemässen Sensor (1), welcher beispielhaft an der Multibracket-Apparatur für den Oberkiefer (2), und somit an den Brackets (23) und/oder dem Bogendraht (24), befestigt ist. Der Sensor (1) erstreckt sich beispielhaft entlang von etwa zwei Zähnen und überdeckt dementsprechend auch zwei Brackets (23). Der Sensor (1) umfasst eine Gummizug-Halterung (16), an welcher der Gummizug (4) befestigt ist. Der Gummizug (4) führt weiter zur Multibracket-Apparatur des Unterkiefers (3), wo er an einem Häkchen eines Brackets (23) angebracht ist. Der Sensor (1) kann natürlich auch an der Multibracket-Apparatur für den Unterkiefer (3) angebracht werden. Bei gestrecktem Gummizug (4) wird dann durch die Gummizug-Halterung (16) ein Druck auf den im Sensor (1) angeordneten Druck-, Zug- und/oder Scherkraftsensor (11) ausgeübt. Dabei wird im Sensor (1) zumindest mit dem Timer die Zeit gemessen und im Datenspeicher des Mikrokontrollers (12) des Sensors (1) gespeichert. Der Timer ist beispielhaft in Form von Software im Mikrokontroller integriert und nicht separat dargestellt. Bei der nächsten Kontrolle beim Behandler können dann die im Datenspeicher des Mikrokontrollers (12) gespeicherten Daten über die im Sensor (1) typischerweise integrierte Datenübertragungsvorrichtung (18) mittels Lesegerät (nicht dargestellt) ausgelesen und anschliessend ausgewertet werden.
Wird der Gummizug (4) nicht oder zu wenig lange getragen, erfolgt keine oder nur eine geringe Justierung der Position vom Oberkiefer zum Unterkiefer. Dies kann durch den Behandler jedoch nur mittels des erfindungsgemässen Sensors (1) und System (5), dem erfindungsgemässen Verfahren sowie der erfindungsgemässen Verwendung objektiv festgestellt werden, da der fehlende Gummizug (4) keinen Druck auf den Sensor (1) ausübt.

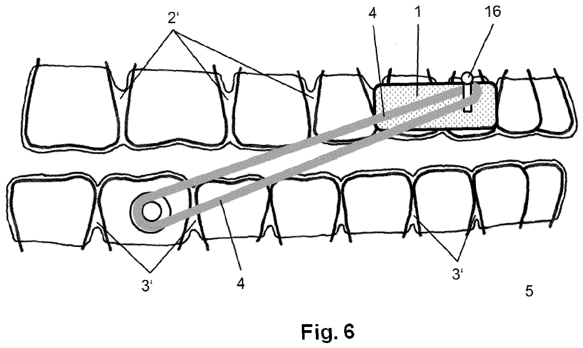

- Fig. 6: zeigt das erfindungsgemässe System (5) umfassend einen Aligner für den Oberkiefer (2') und einen Aligner für den Unterkiefer (3'). Die Aligner sind um die Zähne angeordnet, durch das Zahnfleisch begrenzt und mit einer dünneren Linie dargestellt. Der erfindungsgemässe Sensor (1) ist beispielhaft am Aligner für den Oberkiefer (2') angebracht und erstreckt sich entlang von etwa zwei Zähnen. Der Sensor (1) umfasst eine Gummizug-Halterung (16), an welcher der Gummizug (4) befestigt ist. Der Gummizug (4) führt weiter zum Aligner des Unterkiefers (3'), wo er in dieser Darstellung an einem runden Knöpfchen oder Button befestigt ist. Solche Knöpfchen oder Buttons sind dem Fachmann bekannt und werden typischerweise direkt auf den Zahn geklebt, wobei der Aligner (3') an dieser Stelle eine Aussparung aufweist. Alternativ kann der Gummizug beispielsweise an einer im Aligner (3') angebrachten Kerbe befestigt werden. Der Sensor (1) kann natürlich auch am Aligner für den Unterkiefer (3') und das Knöpfchen oder die Aussparung am Aligner für den Oberkiefer (2') angebracht werden. Bei gestrecktem Gummizug (4) wird dann durch die Gummizug-Halterung (16) ein Druck auf den im Sensor (1) angeordneten Druck-, Zug- und/oder Scherkraftsensor (11) ausgeübt. Dabei wird im Sensor (1) zumindest mittels Timer die Zeit gemessen und im Datenspeicher des Mikrokontrollers (12) des Sensors (1) gespeichert. Periodisch, beispielsweise bei der nächsten Kontrolle beim Behandler, können dann die im Datenspeicher (12) gespeicherten Daten über die im Sensor (1) typischerweise integrierte Datenübertragungsvorrichtung (18) mittels Lesegerät (nicht dargestellt) ausgelesen und anschliessend ausgewertet werden. So kann auf einfache Art und Weise die Tragdauer des Gummizugs (4) bestimmt werden. Dies ist insbesondere bei ungenügender Tragdauer - und somit bei ungenügendem Behandlungserfolg - äusserst hilfreich.

## Patentansprüche

1. Sensor (1) zur Befestigung an einer Multibracket-Apparatur für den Oberkiefer (2), an einer Multibracket-Apparatur für den Unterkiefer (3), an einem Aligner für den Oberkiefer (2') und/oder an einem Aligner für den Unterkiefer (3'), um die Zeitspanne zu messen, während welcher eine Kraft auf einen Druck-, Zug- und/oder Scherkraftsensor (11) des Sensors (1) ausgeübt wird, wodurch die Tragedauer von kieferorthopädischen Gummizügen (4) für Multibracket-Apparaturen (2, 3) und/oder Aligner (2', 3') überwacht werden kann, wobei die Multibracket-Apparaturen eine Vielzahl von Brackets (23) aufweisen, umfassend den Druck-, Zug- und/oder Scherkraftsensor (11), einen Mikrokontroller mit Timer und Datenspeicher (12), ***dadurch gekennzeichnet,* dass** der Sensor (1)
- ein von der Multibracket-Apparatur (2, 3) und den Brackets (23) unabhängiger Sensor ist, sowie
- eine Befestigungsvorrichtung (15) zur Befestigung des Sensors (1) an einer Multibracket-Apparatur (2, 3) und/oder an einem Aligner (2', 3') aufweist, wobei der Sensor (1) anstelle der Befestigungsvorrichtung (15) auch an einem Aligner (2', 3') befestigt sein kann, und
- eine Gummizug-Halterung (16) und/oder Gummizug-Führung (17) umfasst,
wobei der Gummizug (4) an der Gummizug-Halterung (16) des Sensors (1) befestigbar und/oder der Gummizug (4) in der Gummizug-Führung (17) des Sensors (1) führbar ist, wodurch durch den Gummizug (4) eine Kraft auf den Druck-, Zug- und/oder Scherkraftsensor (11) des Sensors (1) wirkt und der Timer die Zeitspanne misst, während welcher die Kraft auf den Sensor (1) wirkt.

2. Sensor (1) nach Anspruch 1, **dadurch gekennzeichnet, dass** der Sensor (1) zudem ein Gehäuse (13), eine Stromversorgung (14) und/oder eine Datenübertragungsvorrichtung (18) umfasst.

3. Sensor (1) nach Anspruch 2, **dadurch gekennzeichnet, dass** die Datenübertragungsvorrichtung (18) einen Funksender und/oder eine Steckverbindung umfasst.

4. Sensor (1) nach mindestens einem der Ansprüche 1 bis 3, **dadurch gekennzeichnet, dass** ein Gummizug (4)
- an der Gummizug-Halterung (16) des Sensors (1) reversibel befestigt werden kann um die Gummizug-Halterung (16) mit einem Bracket (23), Bogendraht (24) oder Aligner (2', 3') zu verbinden, und/oder
- in der Gummizug-Führung (17) des Sensors (1) geführt werden kann, wodurch eine Kraft auf den Druck-, Zug- und/oder Scherkraftsensor (11) ausgeübt wird, wenn der Gummizug (4) von einem Bracket (23) der Multibracket-Apparatur des Oberkiefers (2) oder vom Aligner des Oberkiefers (2') zu einem Bracket (23) der Multibracket-Apparatur des Unterkiefers (3) oder zum Aligner des Unterkiefers (3') gespannt ist.

5. Sensor (1) nach Anspruch 4, **dadurch gekennzeichnet, dass**, wenn der Gummizug (4)
- an der Gummizug-Halterung (16) befestigt ist, die Gummizug-Halterung (16) bei gespanntem Gummizug (4) eine Kraft auf den Druck-, Zug- und/oder Scherkraftsensor (11) des Sensors (1) ausübt, und/oder
- in der Gummizug-Führung (17) geführt wird, der Gummizug (4) anstelle an einem Bracket (23) oder Aligner (2', 3') auch an der Gummizug-Halterung (16) des Sensors (1) befestigt sein kann.

6. Sensor (1) nach mindestens einem der Ansprüche 1 bis 5, **dadurch gekennzeichnet, dass** die Befestigungsvorrichtung (15) eine Öse, Klemme, Schraube, ein Draht, Gummizug, Steckverbindung, Elastik und/oder Klebefläche umfasst, anhand welcher der Sensor (1) im Wesentlichen an jede beliebige Multibracket-Apparatur (2, 3) und an jeden beliebigen Aligner (2', 3') befestigt werden kann.

7. System (5) zur Überwachung der Tragedauer von kieferorthopädischen Gummizügen für Multibracket-Apparaturen (2, 3) und/oder Aligner (2', 3'), umfassend einen Sensor (1) nach mindestens einem der Ansprüche 1 bis 6, eine Multibracket-Apparatur oder einen Aligner für den Oberkiefer (2, 2') und eine Multibracket-Apparatur oder einen Aligner für den Unterkiefer (3, 3'), sowie einen Gummizug (4) und ein externes Lesegerät, wobei die Multibracket-Apparaturen (2, 3) eine Vielzahl von Brackets (23) und je einen Bogendraht (24) umfassen.

8. System (5) nach Anspruch 7, **dadurch gekennzeichnet, dass** die Befestigungsvorrichtung (15) des Sensors (1) nach mindestens einem der Ansprüche 1 bis 6, mit mindestens einem Bracket (23) und/oder dem Bogendraht (24) einer Multibracket-Apparatur (2, 3), und/oder einem Aligner (2', 3'), mit einem Befestigungsmittel verbunden werden kann.

9. System (5) nach Anspruch 8, **dadurch gekennzeichnet, dass** das Befestigungsmittel einen Draht, eine Schraube, eine Klemme, einen Gummizug, einen Elastik, einen Kunststoff und/oder einen Klebstoff oder eine Klebstoffkombination umfasst.

10. System (5) nach mindestens einem der Ansprüche 7 bis 9, **dadurch gekennzeichnet, dass** der Sensor (1) nach mindestens einem der Ansprüche 1 bis 6, wenn er an einer Multibracket-Apparatur (2, 3) befestigt ist,
- zwischen zwei Brackets (23) der gleichen Multibracket-Apparatur (2, 3) angeordnet ist, und/oder
- sich über mindestens ein Bracket (23) der gleichen Multibracket-Apparatur (2, 3) erstreckt.

11. System (5) nach mindestens einem der Ansprüche 7 bis 9, **dadurch gekennzeichnet, dass** der Sensor (1) nach mindestens einem der Ansprüche 1 bis 4, wenn er an einem Aligner (2', 3') befestigt ist,
- im seitlichen Aussenbereich des Aligners (2', 3') angeordnet ist, und/oder
- sich über mindestens eine Zahnkrone des gleichen Aligners (2', 3') erstreckt.

## Claims

1. A sensor (1) for fastening on a multi-bracket apparatus for the upper jaw (2), on a multi-bracket apparatus for the lower jaw (3), on an aligner for the upper jaw (2') and/or on an aligner for the lower jaw (3'), in order to measure the time period during which a force is exerted on a compressive-force, tensile-force and/or shear-force sensor (11) of the sensor (1), as a result of which the wearing time of orthopaedic elastic bands (4) for multibracket apparatuses (2, 3) and/or aligners (2', 3') can be monitored, wherein the multi-bracket apparatuses have a multiplicity of brackets (23), comprising the compressive-force, tensile-force and/or shear-force sensor (11), a microcontroller with timer and data memory (12), **characterized in that** the sensor (1)
- is a sensor independent of the multi-bracket apparatus (2, 3) and the brackets (23), and
- has a fastening device (15) for fastening the sensor (1) on a multi-bracket apparatus (2, 3) and/or on an aligner (2', 3'), wherein the sensor (1) may also be fastened on an aligner (2', 3') instead of the fastening device (15), and
- comprises a elastic-band holder (16) and/or elastic-band guide (17),
wherein the elastic band (4) can be fastened on the elastic-band holder (16) of the sensor (1) and/or the elastic band (4) can be guided in the elastic-band guide (17) of the sensor (1), as a result of which a force acts by means of the elastic band (4) on the compressive-force, tensile-force and/or shear-force sensor (11) of the sensor (1) and the timer measures the time period during which the force acts on the sensor (1).

2. The sensor (1) according to Claim 1, **characterized in that** the sensor (1) additionally comprises a housing (13), a power supply (14) and/or a data transmission device (18).

3. The sensor (1) according to Claim 2, **characterized in that** the data transmission device (18) comprises a radio transmitter and/or a plug connection.

4. The sensor (1) according to at least one of Claims 1 to 3, **characterized in that** a elastic band (4)
- can be fastened in a reversible manner on the elastic-band holder (16) of the sensor (1) in order to connect the elastic-band holder (16) to a bracket (23), arch wire (24) or aligner (2', 3'), and/or
- can be guided in the elastic-band guide (17) of the sensor (1), as a result of which a force is exerted on the compressive-force, tensile-force and/or shear-force sensor (11), if the elastic band (4) is stretched from a bracket (23) of the multi-bracket apparatus of the upper jaw (2) or from the aligner of the upper jaw (2') to a bracket (23) of the multi-bracket apparatus of the lower jaw (3) or to the aligner of the lower jaw (3').

5. The sensor (1) according to Claim 4, **characterized in that**, if the elastic band (4)
- is fastened on the elastic-band holder (16), when the elastic band is stretched (4), the elastic-band holder (16) exerts a force on the compressive-force, tensile-force and/or shear-force sensor (11) of the sensor (1), and/or
- is guided in the elastic-band guide (17), the elastic band (4) can also be fastened on the elastic-band holder (16) of the sensor (1) instead of on a bracket (23) or aligner (2', 3').

6. The sensor (1) according to at least one of Claims 1 to 5, **characterized in that** the fastening device (15) comprises a grommet, clamp, screw, a wire, elastic band, plug connection, elastic and/or adhesive surface, using which the sensor (1) can essentially be fastened on any desired multi-bracket apparatus (2, 3) and on any desired aligner (2', 3').

7. A system (5) for monitoring the wearing time of orthodontic elastic bands for multi-bracket apparatuses (2, 3) and/or aligners (2', 3'), comprising a sensor (1) according to at least one of Claims 1 to 6, a multi-bracket apparatus or an aligner for the upper jaw (2, 2') and a multi-bracket apparatus or an aligner for the lower jaw (3, 3'), and a elastic band (4) and an external reading device, wherein the multi-bracket apparatuses (2, 3) comprise a multiplicity of brackets (23) and one arch wire (24) in each case.

8. The system (5) according to Claim 7, **characterized in that** the fastening device (15) of the sensor (1) according to at least one of Claims 1 to 6, can be connected to at least one bracket (23) and/or the arch wire (24) of a multi-bracket apparatus (2, 3), and/or an aligner (2', 3') using a fastening means.

9. The system (5) according to Claim 8, **characterized in that** the fastening means comprises a wire, a screw, a clamp, a elastic band, an elastic, a plastic and/or an adhesive or an adhesive combination.

10. The system (5) according to at least one of Claims 7 to 9, **characterized in that** the sensor (1) according to at least one of Claims 1 to 6, if it is fastened on a multi-bracket apparatus (2, 3),
- is arranged between two brackets (23) of the same multi-bracket apparatus (2, 3), and/or
- extends over at least one bracket (23) of the same multi-bracket apparatus (2, 3).

11. The system (5) according to at least one of Claims 7 to 9, **characterized in that** the sensor (1) according to at least one of Claims 1 to 4, if it is fastened on an aligner (2', 3'),
- is arranged in the lateral external region of the aligner (2', 3'), and/or
- extends over at least one tooth crown of the same aligner (2', 3').

## Revendications

1. Capteur (1) pour la fixation à un appareil multibracket pour la mâchoire supérieure (2), à un appareil multibracket pour la mâchoire inférieure (3), à un dispositif d'alignement pour la mâchoire supérieure (2') et/ou à un dispositif d'alignement pour la mâchoire inférieure (3') pour mesurer le laps de temps pendant lequel une force s'exerce sur un capteur de force de compression, traction et/ou de cisaillement (11) du capteur (1), moyennant quoi la durée de port de bandes élastiques orthodontiques (4) pour des appareils multibracket (2, 3) et/ou des dispositifs d'alignement (2', 3') peut être surveillée, dans lequel les appareils multibracket présentent une pluralité de brackets (23) comprenant le capteur de force de compression, traction et/ou de cisaillement (11), un microcontrôleur avec une minuterie, et une mémoire de données (12), **caractérisé en ce que** le capteur (1)
- est un capteur indépendant de l'appareil multibracket (2, 3) et des brackets (23), et
- présente un dispositif de fixation (15) pour la fixation du capteur (1) à un appareil multibracket (2, 3) et/ou à un dispositif d'alignement (2', 3'), dans lequel le capteur (1), au lieu d'être fixé au dispositif de fixation (15), peut également être fixé à un dispositif d'alignement (2', 3'), et
- comprend une fixation de cordon élastique (16) et/ou un guidage de cordon élastique (17),
dans lequel la bande élastique (4) peut être fixé à la fixation de cordon élastique (16) du capteur (1) et/ou la bande élastique (4) peut être guidé dans le guidage de cordon élastique (17) du capteur (1), moyennant quoi, du fait de la bande élastique (4), une force s'exerce sur le capteur de force de compression, traction et/ou de cisaillement (11) du capteur (1) et la minuterie mesure le laps de temps pendant lequel la force s'exerce sur le capteur (1).

2. Capteur (1) selon la revendication 1, **caractérisé en ce que** le capteur (1) comprend en outre un boîtier (13), une alimentation électrique (14) et/ou un dispositif de transmission de données (18) .

3. Capteur (1) selon la revendication 2, **caractérisé en ce que** le dispositif de transmission de données (18) comprend un émetteur radio et/ou une connexion par enfichage.

4. Capteur (1) selon l'une au moins des revendications 1 à 3, **caractérisé en ce qu'**une bande élastique (4)
- peut être fixé de manière réversible à la fixation de cordon élastique (16) du capteur (1) pour relier la fixation de cordon élastique (16) à un bracket (23), un arc dentaire (24) ou un dispositif d'alignement (2', 3'), et/ou
- peut être guidé dans le guidage de cordon élastique (17) du capteur (1), moyennant quoi une force s'exerce sur le capteur de force de compression, traction et/ou de cisaillement (11) lorsque la bande élastique (4) est tendu depuis un bracket (23) de l'appareil multibracket de la mâchoire supérieure (2) ou du dispositif d'alignement de la mâchoire supérieure (2') vers un bracket (23) de l'appareil multibracket de la mâchoire inférieure (3) ou vers le dispositif d'alignement de la mâchoire inférieure (3').

5. Capteur (1) selon la revendication 4, **caractérisé en ce que**, lorsque la bande élastique (4)
- est fixé à la fixation de cordon élastique (16), la fixation de cordon élastique (16), en cas de bande élastique a tendu, exerce une force sur le capteur de force de compression, traction et/ou de cisaillement (11) du capteur (1), et/ou
- est guidé dans le guidage de cordon élastique (17), la bande élastique (4), au lieu d'être fixé à un bracket (23) ou à un dispositif d'alignement (2', 3'), pouvant également être fixé à la fixation de cordon élastique (16) du capteur (1).

6. Capteur (1) selon l'une au moins des revendications 1 à 5, **caractérisé en ce que** le dispositif de fixation (15) comprend un œillet, une agrafe, une vis, un fil de fer, un cordon élastique, une connexion par enfichage, un élastique et/ou une surface adhésive à l'aide desquels le capteur (1) peut être fixé sensiblement à chaque appareil multibracket au choix (2, 3) et à chaque dispositif d'alignement (2', 3') au choix.

7. Système (5) pour la surveillance de la durée de port de cordons élastiques orthodontiques pour appareils multibracket (2, 3) et/ou dispositifs d'alignement (2', 3'), comprenant un capteur (1) selon l'une au moins des revendications 1 à 6, un appareil multibracket ou un dispositif d'alignement pour la mâchoire supérieure (2, 2') et un appareil multibracket ou un dispositif d'alignement pour la mâchoire inférieure (3, 3'), ainsi qu'une bande élastique (4) et un appareil de lecture externe, dans lequel les appareils multibracket (2, 3) comprennent une pluralité de brackets (23) et respectivement un arc dentaire (24) .

8. Système (5) selon la revendication 7, **caractérisé en ce que** le dispositif de fixation (15) du capteur (1) selon l'une au moins des revendications 1 à 6 peut être relié avec un moyen de fixation à au moins un bracket (23) et/ou à l'arc dentaire (24) d'un appareil multibracket (2, 3) et/ou à un dispositif d'alignement (2', 3').

9. Système (5) selon la revendication 8, **caractérisé en ce que** le moyen de fixation comprend un fil de fer, une vis, une agrafe, un cordon élastique, un élastique, une matière plastique et/ou un adhésif ou une combinaison d'adhésifs.

10. Système (5) selon l'une au moins des revendications 7 à 9, **caractérisé en ce que** le capteur (1) selon l'une au moins des revendications 1 à 6, lorsqu'il est fixé à un appareil multibracket (2, 3),
- est disposé entre deux brackets (23) du même appareil multibracket (2, 3) et/ou
- s'étend sur au moins un bracket (23) du même appareil multibracket (2, 3).

11. Système (5) selon l'une au moins des revendications 7 à 9, **caractérisé en ce que** le capteur (1) selon l'une au moins des revendications 1 à 4, lorsqu'il est fixé à un dispositif d'alignement (2', 3'),
- est disposé dans la zone extérieure latérale du dispositif d'alignement (2', 3'), et/ou
- s'étend sur au moins une couronne dentaire du même dispositif d'alignement (2', 3').
